Europäisches Patentamt
European Patent Office
Office européen des brevets

(19) (11) Veröffentlichungsnummer: **0 351 696 A2**

(12) **EUROPÄISCHE PATENTANMELDUNG**

(21) Anmeldenummer: **89112687.2**

(22) Anmeldetag: **11.07.89**

(51) Int. Cl.4: **C07D 233/61 , C07D 249/08 , C07D 409/06 , C07D 409/12 , C07D 409/14 , A01N 43/50 , A01N 43/653**

Patentansprüche für folgende Vertragsstaaten: ES + GR.

(30) Priorität: **22.07.88 AT 1875/88**

(43) Veröffentlichungstag der Anmeldung: **24.01.90 Patentblatt 90/04**

(84) Benannte Vertragsstaaten: **AT BE CH DE ES FR GB GR IT LI LU NL SE**

(71) Anmelder: **CL PHARMA AKTIENGESELLSCHAFT**
**St. Peter-Strasse 25**
**A-4021 Linz(AT)**

(72) Erfinder: **Schermanz, Karl, Dr.**
**Gaussgasse 4**
**A-8010 Graz(AT)**
Erfinder: **Saischek, Gerald, Dipl.-Ing.**
**Industriestrasse 10**
**A-8502 Lannach(AT)**
Erfinder: **Kores, Dietmar, Dr.**
**Hochstrasse 6a**
**A-4060 Leonding(AT)**
Erfinder: **Graf, Josef, Ing.**
**Kürnberg 117**
**A-4442 Kleinraming(AT)**
Erfinder: **Haas, Gerhard**

**A-4692 Niederthalheim(AT)**
Erfinder: **Martetschläger, Kurt**
**Himmelbergerstrasse 14**
**A-4020 Linz(AT)**

(74) Vertreter: **Kunz, Ekkehard, Dr.**
**Chemie Holding AG Patentwesen St. Peter-Strasse 25**
**A-4021 Linz(AT)**

(54) **Allylaminoethylazole.**

(57) Neue Allylaminoethylazole der allgemeinen Formel I

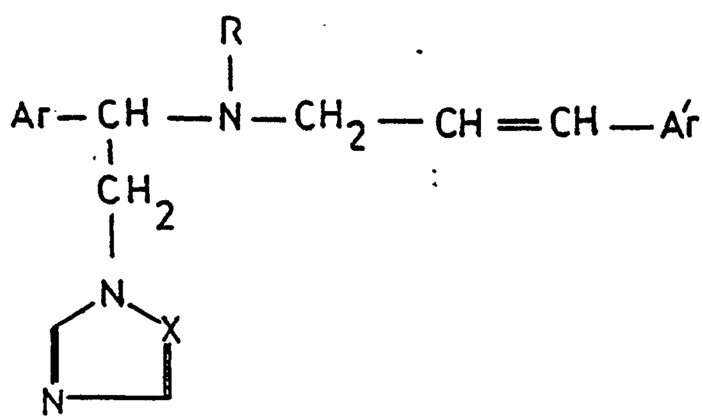

I

in der X ein Stickstoffatom oder eine CH-Gruppe, R ein Wasserstoffatom oder eine Alkylgruppe eine



Xerox Copy Centre

Alkylgruppe (1 bis 5 C-Atome) und Ar und Ar′ unabhängig voneinander einen unsubstituierten, ein- oder mehrfach durch Alkyl (1 bis 3 C-Atome), Alkoxy (1 bis 3 C-Atome), Nitrogruppen oder Halogen substituierten Phenylrest, einen unsubstituierten oder durch Halogen substituierten Thienylrest oder einen Naphthylrest bedeuten, sowie deren pflanzenphysiologisch oder pharmakologisch verträglichen Säureadditionssalze, Verfahren zur Herstellung dieser neuen Verbindungen und ihre Verwendung als fungizide oder antimykotische Mittel.

## Neue Allylaminoethylazole

Die Erfindung betrifft neue Allylaminoethylazole, Verfahren zu ihrer Herstellung sowie diese enthaltende fungizide und antimykotische Mittel, Verfahren zu deren Herstellung und deren Verwendung.

Aus EP-A 61.798 wurden substituierte 2-Phenyl-ethyl-amino-1,2,4-triazole bekannt, die fungizide Eigenschaften aufweisen. Das Bauprinzip der Allylaminoethylazole wurde nicht erwähnt. Unerwarteterweise wurden nun neue Allylaminoethylazole gefunden, die sich durch starke fungizide und antimykotische Wirkung auszeichnen.

Gegenstand der Erfindung sind daher neue Allylaminoethylazole der allgemeinen Formel I des Formelblattes in der

X ein Stickstoffatom oder eine CH-Gruppe

R ein Wasserstoffatom oder eine Alkylgruppe mit 1 bis 5 C-Atomen und

Ar und Ar′ unabhängig voneinander einen unsubstituierten, ein- oder mehrfach durch Alkyl (1 bis 3 C-Atome), Alkoxy (1 bis 3 C-Atome), Nitrogruppen oder Halogen substituierten Phenylrest, einen unsubstituierten oder durch Halogen substituierten Thienylrest oder einen Naphthylrest bedeuten sowie deren pflanzenphysiologisch oder pharmakologisch verträglichen Säureadditionssalze.

In der Formel I bedeutet X ein Stickstoffatom oder eine CH-Gruppe, bevorzugt eine CH-Gruppe, R ein Wasserstoffatom oder eine Alkylgruppe mit 1 bis 5 C-Atomen, bevorzugt ein Wasserstoffatom und Ar und Ar′ unabhängig voneinander einen unsubstituierten, ein- oder mehrfach durch Alkyl-(1 bis 3 C-Atome), Alkoxy-(1 bis 3 C-Atome), Nitrogruppen oder Halogen substituierten Phenylrest, einen unsubstituierten oder durch Halogen substituierten Thienylrest oder einen Naphthylrest, vorzugsweise einen unsubstituierten oder ein- oder mehrfach durch Halogen substituierten Phenylrest.

Insbesondere bedeutet Ar bevorzugt einen durch Halogen substituierten Phenylrest und abhängig davon Ar′ vorzugsweise einen Phenylrest, der gegebenenfalls durch Alkyl (1 bis 3 C-Atome), Alkoxy (1 bis 3 C-Atome) oder auch durch Halogen substituiert sein kann.

Unter dem Begriff Alkyl ist je nach der Anzahl der angegebenen Kohlenstoffatome beispielsweise eine der folgenden Gruppen zu verstehen: Methyl, Ethyl, Propyl, Butyl, Pentyl usw. sowie ihre Isomeren wie beispielsweise Isopropyl, Isobutyl, tert.-Butyl, sek.-Butyl, Isopentyl usw.

Unter dem Begriff Alkoxy ist je nach Anzahl der angegebenen Kohlenstoffatome beispielsweise eine Methoxy-, Ethoxy-, Propoxy- oder iso-Propoxy-Gruppe zu verstehen.

Halogen bedeutet Fluor, Chlor, Brom und Jod, insbesondere Chlor.

Ganz besonders bevorzugt sind die Verbindungen

1-(2-(2,4-Dichlorphenyl)-2-(3-phenylallylamino))ethyl-1H-imidazol

1-(2-(2,4-Dichlorphenyl)-2-(3-(4-methoxyphenyl)allylamino))ethyl-1H-imidazol

1-(2-(2,4-Dichlorphenyl)-2-(3-(4-methylphenyl)allylamino))ethyl-1H-imidazol

1-(2-(2,4-Dichlorphenyl)-2-(3-(2-chlorphenyl)allylamino))ethyl-1H-imidazol

1-((2-(2,4-Dichlorphenyl)-2-(3-(4-chlorphenyl)allylamino))ethyl-1H-triazol

Die neuen Verbindungen können auch in Form ihrer Säureadditionssalze oder Metallkomplexe vorliegen.

Als Säureadditionssalze kommen Salze anorganischer oder organischer Säuren, beispielsweise Hydrochloride, -bromide und -jodide, Nitrate, Hydrogensulfate, Methosulfate, Trifluormethylsulfonate, Tosylate, Acetate, Trifluoracetate, Lactate, Malate und Benzoate in Frage.

Ein weiterer Gegenstand der Erfindung ist ein Verfahren zur Herstellung der neuen Allylaminoethylazole der allgemeinen Formel I des Formelblattes in der X, R, Ar und Ar′ die im Anspruch 1 genannte Bedeutung haben, dadurch gekennzeichnet, daß man

a) eine Verbindung der allgemeinen Formel II des Formelblattes, in der X und Ar die obgenannte Bedeutung haben, mit einer Verbindung der allgemeinen Formel III des Formelblattes, in der Ar′ die obgenannte Bedeutung hat, gegebenenfalls in einem inerten Verdünnungsmittel zu einer Verbindung der allgemeinen Formel IV des Formelblattes oder

b) eine Verbindung der allgemeinen Formel V des Formelblattes, in der X und Ar die obgenannte Bedeutung haben, mit einer Verbindung der allgemeinen Formel VI des Formelblattes, in der Ar′ die obgenannte Bedeutung hat, gegebenenfalls in einem inerten Verdünnungsmittel zu einer Verbindung der allgemeinen Formel VII des Formelblattes umsetzt und anschließend die gebildete Verbindung der allgemeinen Formel IV des Formelblattes oder die gebildete Verbindung der allgemeinen Formel VII des Formelblattes in Gegenwart eines inerten Verdünnungsmittel durch Zugabe eines Reduktionsmittels zu einer Verbindung der allgemeinen Formel I des Formelblattes, in der R ein Wasserstoffatom bedeutet, reduziert und gewünschtenfalls zu einer Verbindung der allgemeinen Formel I des Formelblattes, in der R einen Alkylrest

(1 bis 5 C-Atome) bedeutet, alkyliert.

Die Umsetzung der Verbindungen der Formel II mit den Verbindungen der Formel III bzw. die Umsetzung der Verbindungen der Formel V mit den Verbindungen der Formel VI erfolgt zweckmäßig in einem organischen Verdünnungsmittel.

Als Verdünnungsmittel dienen aliphatische oder aromatische Kohlenwasserstoffe, die chloriert sein können, wie Benzinfraktionen, Perchlorethylen, Benzol, Toluol, Chlorbenzol, Xylol, Ether wie Dibutylether, Dioxan, Alkohole wie Butanol, Pentanol, Ethylenglykol, Säureamide wie Dimethylformamid und Mischungen derselben mit den oben genannten Verdünnungsmittel. Vorzugsweise verwendet man Toluol.

Die Reaktion wird bei Temperaturen von etwa 0 bis 180 °C ausgeführt, bevorzugt bei Siedetemperatur des jeweiligen Verdünnungsmittels. Das gebildete Reaktionswasser entfernt man vorteilhafterweise durch azeotrope Destillation. Im allgemeinen setzt man die Ausgangsstoffe in stöchiometrischem Verhältnis ein. Ein Überschuß des einen oder anderen kann im Einzelfalle aber durchaus von Vorteil sein. In einer bevorzugten Ausführungsform werden die Ausgangsprodukte in stöchiometrischem Verhältnis in Toluol gelöst oder suspendiert. Man erhitzt sodann unter Verwendung eines Wasserabscheiders auf Rückfluß, bis sich kein Reaktionswasser mehr abscheidet.

Die entstandenen Iminoverbindungen der Formel IV bzw. VII werden nach Entfernen des Verdünnungsmittels ohne weitere Reinigung zu den Verbindungen der Formel I, in der R ein Wasserstoffatom bedeutet, reduziert. Dazu löst oder suspendiert man die Iminoverbindungen in einem organischen Verdünnungsmittel und setzt ein Reduktionsmittel zu. Als Verdünnungsmittel können je nach Art des verwendeten Reduktionsmittels Alkohole wie Methanol, Ethanol, etc. oder Ether wie Diethylether, Diisopropylether oder Tetrahydrofuran dienen.

Als Reduktionsmittel kommen beispielsweise komplexe Metallhydride wie Natrium-, Lithium- oder Aluminiumborhydrid, Natrium- oder Lithiumcyanoborhydrid, Lithiumaluminiumhydrid etc. in Betracht, es ist aber auch möglich, die Hydrierung mit Wasserstoff in Gegenwart geeigneter Katalysatoren durchzuführen. Vozugsweise führt man die Reduktion mit Natriumborhydrid in Methanol aus, wobei das Metallhydrid üblicherweise in einem 1,1 bis 20fachen Überschuß eingesetzt wird. Die Reaktion kann bei Temperaturen zwischen etwa -20 °C bis zur Siedetemperatur des verwendeten Lösungsmittels durchgeführt werden, vorzugsweise arbeitet man zwischen -5 °C und 65 °C.

Nach beendeter Reaktion wird das überschüssige Metallhydrid durch Zugabe einer Säure, vorzugsweise Salzsäure, zerstört, wobei gegebenenfalls das Lösungsmittel vor oder nach der Säurezugabe entfernt wird. Zur weiteren Aufarbeitung wird durch Zusetzen von Alkali, vorzugsweise wäßriger Natronlauge, die Base der Verbindung IV oder VII freigesetzt und mit Hilfe eines organischen, mit Wasser nicht mischbaren, Lösungsmittels, wie Essigsäurethylester oder Dichlormethan extrahiert. Nach Trocknen und Entfernen des organischen Lösungsmittels kann man die Verbindung der Formel I, in der R ein Wasserstoffatom bedeutet unter Benutzung üblicher Alkylierungsmethoden in eine Verbindung der Formel I überführen, in der R einen Alkylrest (1 bis 5 C-Atomen) bedeutet.

Man kann dazu beispielsweise Alkylierungsreagentien der allgemeinen Formel RY benutzen, in der R einen Alkylrest und Y eine Abgangsgruppe wie z. B. Chlorid, Bromid, Jodid oder eine Benzylsulfonyl-, Toluolsulfonyl- oder Methansulfonylgruppe usw. bedeutet. Zur Einführung eines Methylrestes kann man auch die Verbindung der Formel I, in der R ein Wasserstoffatom bedeutet, in einer wäßrigen Formaldehydlösung in Gegenwart eines Reduktionsmittels wie z. B. Ameisensäure umsetzen.

Die Reinigung der Verbindungen der Formel I des Formelblattes kann durch Umkristallisieren, Säulenchromatographie oder durch Bildung und Ausfällung der Säureadditionssalze erfolgen. Zur Bildung der Säureadditionssalze löst man die Verbindungen der Formel I in einem organischen Lösungsmittel wie z. B. Diethylether, Essigsäureethylester, Aceton oder iso-Propylalkohol und fällt durch Zugabe von anorganischer oder organischer Säure das entsprechende Säureadditionssalz aus, das isoliert und gegebenenfalls aus einem organischen Lösungsmittel wie Essigsäureethylester, iso-Propanol, Ethanol usw. umkristallisiert wird.

Die Ausgangsstoffe der Formel II sind bekannt und können beispielsweise nach Drug Research 29 (II), Nr. 10, 1511 (1979) oder J. Med. Chem., Vol. 24, 67 (1981), hergestellt werden. Die Überführung der Ketone der Formel II in die Amine der Formel V gelingt beispielsweise nach J. Med. Chem. 12, 790 (1969) und J. Med. Chem. 18, 531 (1975).

Die Ausgangsverbindungen der Formel III des Formelblattes können z. B. nach Robert Walter jun., J. Am. Chem. Soc. 74, 5185 (1952) aus den entsprechenden Zimtaldehyden hergestellt werden. Substituierte Zimtaldehyde, Verbindungen der Formel VI können beispielsweise nach Straus, Liebig's Annalen d. Chemie, 393, 311, 1858 dargestellt werden.

Die erfindungsgemäßen Allylaminoethylazole und ihre Salze zeigen ausgezeichnete fungizide Wirkung und stellen somit eine Bereicherung der Technik dar. Sie sind gegen ein breites Spektrum pflanzenpathogener Pilze wirksam, z. B. gegen Oomyceten, Ascomyceten, Basidiomyceten und Deuteromyceten.

Die gute Pflanzenverträglichkeit und die systemische Wirkungsweise in der zur Behandlung von Pflanzenkrankheiten notwendigen Konzentration, erlaubt eine Behandlung von oberirdischen Pflanzenteilen, von Pflanz- und Saatgut.

Als Pflanzen seien etwa Getreidearten wie Weizen, Gerste, Roggen oder Hafer, weiters Mais, Erdbeeren, Zierpflanzen, Kartoffeln sowie Gemüsearten wie Gurken, Bohnen oder Tomaten genannt.

Mit besonders gutem Erfolg können die erfindungsgemäßen Mittel etwa zur Bekämpfung folgender Pflanzenkrankheiten eingesetzt werden:
Botryotinia fuckeliana (Grauschimmel) an Erdbeeren
Cercospora beticola (Rübenblattfleckenkrankheit) an Rüben
Pseudocercosporella herpotrichoides (Halmbruch) an Getreide
Cochliobolus sativus (Helminthosporiose) an Getreide
Pyrenophora teres (Netzfleckenkrankheit) an Gerste
Phaeosphaeria nodorum (Braunspelzigkeit, Blattdürre) an Getreide
Septoria apiicola (Sellerieblattfleckenkrankheit) an Sellerie
Venturia inaequalis (Schorferkrankung) an Äpfeln
Fusarium culmorum (Halmbasiserkrankung) an Getreide
Alternaria alternata (Schwärzepilz) an Getreide
Aspergillus niger (Schwarzer Gießkannenschimmel)
Monographella nivalis (Schneeschimmel) an Getreide

Die neuen Wirkstoffe können je nach ihrem Anwendungsgebiet in die üblichen Formulierungen übergeführt werden, wie Lösungen, Spritzpulver, Emulsionskonzentrate, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.a., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von Tensiden, also Emulgatoren und/oder Dispergier, und/oder Netzmitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Cyclohexan oder Paraffine, z. B. Erdölfraktionen, Alkohole, wie Butanol oder Glykol, sowie deren Ether und Ester, Ketone, wie Aceton, Methylethylketon, Methylisobutylketon oder Cyclohexanon, stark polare Lösungsmittel, wie Dimethlyformamid und Dimethylsulfoxid, sowie Wasser. Mit verflüssigten, gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe, sowie Butan, Propan, Stickstoff und Kohlendioxid; als feste Trägerstoffe kommen in Frage: z. B. natürliche Gesteinsmehle, wie Kaoline, Tonerde, Talkum, Kreide, Quarz, Attapulgit, Montmorillonit oder Diatomeenerde und synthetische Produkte, wie hochdisperse Kieselsäure, Aluminiumoxid und Silikate; als feste Trägerstoffe für Granulate kommen in Frage: z. B. gebrochene und fraktionierte natürliche Gesteine, wie Calcit, Marmor, Bims, Sepiolith, Dolomit sowie synthetische Granulate aus anorganischen und organischen Mehlen, sowie Granulate aus organischem Material, wie Sägemehl, Kokosnuss-Schalen, Maiskolben und Tabakstengel; als Emulgiermittel und/oder schaumerzeugenden Mittel kommen in Frage: z. B. nichtionogene und ionogene Tenside, wie Polyoxyethylen-Sorbitan-Tallölester, Na-Oleylmethyltaurid, Polyoxyethylen-Fettsäure-Ester, Polyoxyethylen-Fettalkoholether, z. B. Alkylarylpolyglykol ether, Alkylsulfonate, Alkylsulfate, Arylsulfate und Arylalkylsulfonate sowie Eiweisshydrolysate; als Dispergiermittel kommen in Frage: z. B. Ligninsulfonate oder Kondensationsprodukte von Arylsulfonaten mit Formaldehyd.

Es können in den Formulierung Haft- und Verdickungsmittel wie Carboxymethylcellulose, Methylcellulose, natürliche und synthetische pulverige, körnige und latexförmige Polymere verwendet werden, wie Gummi arabicum, Polyvinylalkohol, Polyvinylacetat.

Es können Farbstoffe, wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen erhalten im allgemeinen zwischen 0,1 und 95 Gew.% Wirkstoff, vorzugsweise zwischen 0,5 und 50 %.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch

Gießen, Tauchen, Spritzen, Sprühen, Vernebeln, Verdampfen, Injizieren, Verschlämmen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.%, vorzugsweise zwischen 0,5 und 0,001 %.

Bei der Anwendung der Wirkstoffe zur Behandlung von Pilzinfektionen liegen die Aufwandmengen zwischen 0,015 und 4 kg Wirkstoff/ha Fläche.

Zum Oberflächenschutz von Bäumen und Früchten können die Wirkstoffe auch in Verbindung mit Kunststoffdispersionen 0,25%ig bis 5%ig, bezogen auf das Gewicht der Dispersion, verwendet werden.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.% vorzugsweise 0,0001 bis 0,02 %, am Wirkungsort erforderlich.

Weiters hat sich unerwarteterweise gezeigt, daß die neuen Verbindungen der allgemeinen Formel I des Formelblattes, insbesonders die Verbindungen in der X eine CH-Gruppe bedeutet und ihre Säureadditionssalze auch hervorragende antimykotische Eigenschaften aufweisen. Sie besitzen eine ausgezeichnete Wirksamkeit gegen Erreger, die fungizide Krankheiten an Tier und Mensch auslösen, wie beispielsweise gegen Hefen, Schimmelpilze und Dermatophyten und können somit als Heilmittel, z. B. in Form pharmazeutischer Präparate Verwendung finden, welche die erfindungsgemäßen Verbindungen in Mischung mit einem für die topische, enterale oder parenterale Applikation geeigneten pharmazeutischen organischen oder anorganischen Trägermaterial enthalten.

Pharmazeutisch verträgliche, nicht-toxische Träger oder Exzipientien, die normalerweise für feste Formulierungen verwendet werden, sind Tricalciumphosphat, Calciumcarbonat, Kaolin, Bentonit, Talkum, Gelatine, Lactose, Stärke und dergleichen; für halbfeste Formulierungen seien beispielsweise Polyalkylenglykole, Vaseline, Petrolatum und andere Salbengrundlagen genannt; für flüssige Formulierungen können beispielsweise Wasser, pflanzliche Öle und niedrigsiedende Lösungsmittel wie Isopropanol, hydrierte Naphthaline und dergleichen verwendet werden.

Die pharmazeutischen Präparate können in fester Form z. B. als Tabletten, Dragees, Suppositorien, Kapseln, in flüssiger Form z. B. als Lösungen, Suspensionen oder Emulsionen oder in halbflüssiger Form z. B. als Cremes, Lotionen, Gels oder Salben vorliegen. Gegebenenfalls sind sie sterilisiert und/oder enthalten Hilfsstoffe, wie Konservierungs-, Stabilisierungs- oder Emulgierungsmittel, Salze zur Veränderung des osmotischen Druckes oder Puffer. Sie können auch in Kombination mit anderen therapeutisch wertvollen Stoffen verabreicht werden.

Die Formulierungen enthalten im allgemeinen zwischen 0,1 und 99,9 Gew.% Wirkstoff.

Bei topischen Formulierungen kann die Menge beispielsweise ungefähr 0,1 bis 10 % der gesamten pharmazeutischen Formulierung betragen, wogegen bei anderen Formulierungen die Menge ungefähr 5 bis ungefähr 95 % oder mehr ausmachen kann.

Bei Anwendung in der Pharmazie ist die topische Applikation bevorzugt. Dazu kann eine Fläche, die mit Pilzen oder Bakterien befallen ist oder die gegen Pilz-oder Bakterienbefall geschützt werden soll, mit den erfindungsgemäßen Verbindungen oder Mitteln, die diese enthaltenden, beispielsweise durch Bepudern, Beträufeln, Besprayen, Spülen, Bürsten, Baden, Bestreichen, Überziehen, Imprägnieren und ähnliches, behandelt werden.

Die genaue Vorschrift zur pharmazeutischen Applikation der erfindungsgemäßen Verbindungen und Mittel hängt notwendigerweise von den Anforderungen des Einzelfalles, der Art der Behandlung, die beispielsweise vorbeugend oder heilend sein kann, der Art der beteiligten Organismen und natürlich vom Urteil des behandelnden Arztes ab.

## Beispiel 1

### 1-(2-(2,4-Dichlorphenyl)-2-(3-phenylallylamino))ethyl-1H-imidazol

#### a) 1-(2-(2,4-Dichlorphenyl)-2-(3-phenylallylimino)ethyl-1H-imidazol

12,34 g (0.048 Mol) 2,4-Dichlorphenacylimidazol und 6,66 g (0.05 Mol) Cinnamylamin wurden in 100 ml Toluol suspendiert und unter Verwendung eines Wasserabscheiders solange auf Rückfluß erhitzt, bis kein

Reaktionswasser mehr abgeschieden wurde. Das Lösungsmittel wurde im Vakuum entfernt, wonach 17,4 g (98 % der Theorie) eines viskosen Öles erhalten wurden.

b) 1-(2-(2,4-Dichlorphenyl)-2-(3-phenylallylamino))ethyl-1H-imidazol

17,4 g (0.047 Mol) des Produktes aus Stufe a) wurden in ca. 400 ml Methanol gelöst, die Lösung auf 0 °C gekühlt und 6,5 g (0.17 Mol) Natriumborhydrid portionsweise so eingebracht, daß die Temperatur nicht über 5 °C anstieg. Nach Entfernung der Kühlung wurde 2 Stunden bei 20 bis 30 °C nachgerüht, das Lösungsmittel abgedampft und der gallertige Rückstand mit 150 ml halbkonzentrierter Salzsäure versetzt, um überschüssiges Natriumborhydrid zu vernichten. Anschließend wurde mit 40%iger, wäßriger Natronlauge ein pH von 13 bis 14 eingestellt und die wäßrige, basische Lösung mehrmals mit Dichlormethan extrahiert, die organische Phase mit Wasser gewaschen, getrocknet und abgedampft.

Der ölige Rückstand wurde in Aceton aufgenommen und mit konzentrierter Salpetersäure versetzt, wobei das Dinitrat der Verbindung kristallin ausfiel.
Ausbeute: 11,2 g (49 % der Theorie)
Schmelzpunkt: 190 - 192 °C

## Beispiel 2

1-(2-(2,4-Dichlorphenyl)-2-(3-(4-chlorphenyl)allylamino))ethyl-1H-1,2,4-triazol

a) 1-(2-(2,4-Dichlorphenyl)-2-(3-(4-chlorphenyl)-2-propenylidenimino))ethyl-1H-1,2,4-triazol

9,0 g (0.035 Mol) 1-(2-Amino-2-(2,4-dichlorphenyl))ethyl-1H-1,2,4-triazol und 5,83 g (0.035 Mol) 4-Chlorzimtaldehyd wurden in 80 ml Toluol suspendiert und unter Verwendung eines Wasserabscheiders solange auf Rückfluß erhitzt, bis kein Reaktionswasser mehr abgeschieden wurde. Nach Entfernung des Lösungsmittels im Vakuum wurde der ölige Rückstand in Methanol ausgerührt, wobei 14 g (98,5 % der Theorie) kristallines Produkt mit einem Schmelzpunkt von 144 bis 147 °C erhalten wurden.

b) 1-(2-(2,4-Dichlorphenyl)-2-(3-(4-chlorphenyl)allylamino))ethyl-1H-1,2,4-triazol

11,8 g (0.029 Mol) des Produktes aus Stufe a) wurden in 500 ml Methanol suspendiert, die Suspension auf 0 °C gekühlt und 3,78 g (0.1 Mol) Natriumborhydrid portionsweise so eingebracht, daß die Temperatur nicht über 5 °C anstieg, wobei eine klare Lösung entstand. Nach Entfernung der Kühlung wurde noch etwa 1 Stunde bei 20 bis 30 °C nachgerührt, das Lösungsmittel im Vakuum entfernt und zur Zerstörung des überschüssigen Natriumborhydrides mit halbkonzentrierter Salzsäure versetzt. Anschließend wurde durch Zugabe von 40%iger, wäßriger Natronlauge ein pH-Wert von 13 bis 14 eingestellt, die wäßrige, basische Lösung mehrmals mit Dichlormethan extrahiert, die organische Phase mit Wasser gewaschen, getrocknet und abgedampft. Der ölige Rückstand wurde in Aceton aufgenommen und mit konzentrierter Salpetersäure versetzt, wobei das Dinitrat der Verbindung kristallin ausfiel.
Ausbeute: 10,9 g (80 % der Theorie)
Schmelzpunkt: 195 - 197 °C

Nach einer der obigen Verfahrensweise wurden, unter Verwendung der entsprechenden Ausgangsstoffe, die in der Tabelle aufgeführten Verbindungen 3 bis 27 hergestellt.

| Nr. | Ar | X | R | Ar′ | Salz | Fp (°C) |
|---|---|---|---|---|---|---|
| 3 | 2,4-Dichlorphenyl | CH | Methyl | Phenyl | $2HNO_3$ | 135-137 |
| 4 | 2,4-Dichlorphenyl | CH | H | 4-Tolyl | $2HNO_3$ | 170-176 |
| 5 | 2,4-Dichlorphenyl | CH | H | 4-Methoxyphenyl | $2HNO_3$ | 168-173 |
| 6 | 2,4-Dichlorphenyl | CH | H | 2-Chlorphenyl | $2HNO_3$ | 170-195 |
| 7 | 2,4-Dichlorphenyl | CH | H | 4-Chlorphenyl | $2HNO_3$ | 184-190 |
| 8 | 2,4-Dichlorphenyl | CH | Methyl | 4-Chlorphenyl | Base | Öl |
| 9 | 2,4-Dichlorphenyl | CH | H | 2,4-Dichlorphenyl | $2HNO_3$ | 190-211 |
| 10 | 1-Naphthyl | CH | H | Phenyl | $2HNO_3$ | 166-168 |
| 11 | 2-Thienyl | CH | H | Phenyl | Base | Öl |
| 12 | 2,4-Dichlorphenyl | CH | Propyl | Phenyl | Base | Öl |
| 13 | 2,4-Dichlorphenyl | N | H | Phenyl | $2HNO_3$ | 139-149 |
| 14 | 2,4-Dichlorphenyl | N | Methyl | Phenyl | $2HNO_3.H_2O$ | 125-132 |
| 15 | 2,4-Dichlorphenyl | N | H | 4-Tolyl | $2HNO_3$ | 165-173 |
| 16 | 2,4-Dichlorphenyl | N | H | 4-Methoxyphenyl | $2HNO_3$ | 163-167 |
| 17 | 2,4-Dichlorphenyl | N | H | 2-Chlorphenyl | $2HNO_3$ | 174-185 |
| 18 | 2,4-Dichlorphenyl | N | Methyl | 2-Chlorphenyl | $2HNO_3$ | 119-124 |
| 19 | 2,4-Dichlorphenyl | N | H | 4-Chlorphenyl | Base | 77-110 |
| 20 | 2,4-Dichlorphenyl | N | Methyl | 4-Chlorphenyl | $2HNO_3$ | 137-142 |
| 21 | 2,4-Dichlorphenyl | N | H | 2,4-Dichlorphenyl | $HNO_3$ | 184-220 |
| 22 | 2,4-Dichlorphenyl | N | Methyl | 2,4-Dichlorphenyl | Base | Öl |
| 23 | 2,4-Dichlorphenyl | N | H | 2,6-Dichlorphenyl | $HNO_3$ | 167-179 |
| 24 | 2,4-Dichlorphenyl | N | H | 3,4-Dichlorphenyl | $HNO_3$ | 181-204 |
| 25 | 2,4-Dichlorphenyl | N | H | 2-Nitrophenyl | $HNO_3$ | 193-205 |
| 26 | 2,4-Dichlorphenyl | N | H | 3-Nitrophenyl | $2HNO_3.H_2O$ | 173-178 |
| 27 | 2,4-Dichlorphenyl | N | H | 4-Brom-2-thienyl | $2HNO_3$ | 123-147 |

Beispiel 28

Emulgierbares Konzentrat

10 % Wirkstoff der Verbindung Nr. 3
25 % 4-Butyrolacton
55 % Xylol
10 % Atlox 3335-B

Beipsiel 29

Emulgierbares Konzentrat

25 % Wirkstoff der Verbindung Nr. 2
20 % Ethoxyliertes Dinonylphenol (40 Mol Ethylenoxid/Mol Nonylphenol)
65 % Dimethylsulfoxid

Aus diesen Konzentraten können mit Wasser Emulsionen jeder gewünschten Konzentration hergestellt werden.

Beispiel 30

Spritzpulver

20 % Wirkstoff der Verbindung Nr. 1
50 % Kaolin
20 % Wessalon "SV"
10 % Arkopon $T^R$

Beispiel 31

Spritzpulver

50 % Wirkstoff der Verbindung No. 2
30 % Kaolin
10 % Wessalon "SV"
10 % Arkopon $T^R$

Die Wirkstoffe wurden in einem Mischer mit den Zuschlagsstoffen innig vermischt und anschließend über Walzen und Mühlen vermahlen, wodurch ein Spritzpulver von vorzüglicher Benetzbarkeit erhalten wurde, das mit Wasser zu Suspensionen jeder gewünschten Konzentration verdünnbar ist.

Die folgenden Testbeispiele A bis G betreffen die neuen Allylaminoazole als Fungizide in der Landwirtschaft.

Beispiel A

In vitro Prüfungen

a) Abgekühlte Nährböden wurden mit einer wäßrigen Suspension von Hyphenteilen und/oder Konidiosporen der jeweiligen Testpilze beimpft. Filterplättchen (Durchmesser 5 mm) wurden in wäßrigen 0.001 bis 0.2%igen. Dispersionen der Wirkstofformulierungen getränkt und auf die beimpften Nährböden gelegt. Zur Beurteilung wurde die minimale Hemmkonzentration (MHK) herangezogen. Das ist jene Konzentration des Wirkstoffes in der Dispersion, die das Wachstum des Pilzes vollständig verhindert.

Als Standards wurden
A Thiabendazol: 2-(4-Thiazolyl)-benzimidazol
B Propiconazol: 1-(2-(2,4-Dichlorphenyl)-4-propyl-1,3-dioxolan-2-ylmethyl)-1H-1,2,4-triazol
C Prochloraz: 1-(N-propyl-N-(2-(2,4,6-trichlorphenoxy)-ethyl-carbamoyl)imidazol
verwendet.

Die Zusammensetzung der verwendeten Nährböden war folgende:
Nährboden I: Pepton, Biomalz, Agar.
Nährboden II: Haferflockenauszug, Agar.
Nährboden III: Karottenextrakt, Kartoffelextrakt, Agar.
Nährboden IV: Biomalz, Agar.
Nährboden V: Hefeextrakt, Glukose, Agar.
Nährboden VI: Pepton, Biomalz, Agar.

## Ergebnisse

| | MHK (ppm a.i) | Nährboden |
|---|---|---|
| 1) Alternaria alternata | | |
| Standard C | 10 | II |
| Verbindung 1 | 10 | |
| 2) Aspergillus niger | | |
| Standard A | 100 | I |
| Verbindungen 1, 6 | 25 | |
| Verbindungen 5, 7 | 50 | |
| 3) Botryotinia fuckeliana | | |
| Standard A | 50 | I |
| Verbindung 1 | 10 | |
| Verbindungen 4, 7, 15 | 25 | |
| 4) Cercospora beticola | | |
| Standard C | 5 - 10 | III |
| Verbindungen 2, 3, 5, 6, 19, 20, 27 | 1 | |
| Verbindungen 1, 4, 15, 16, 17 | 2, 5 | |
| Verbindungen 9, 14 | 5 | |
| Verbindung 4 | 10 | |
| 5) Cochliobolus sativus | | |
| Standard C | 10 | IV |
| Verbindung 19 | 5 | |
| Verbindungen 1, 2, 13 | 10 | |
| 6) Fusarium culmorum | | |
| Standard A | 100 | |
| Verbindungen 1, 3, 4, 5, 6 | 25 | |

| | | | |
|---|---|---|---|
| 7) Fusarium oxysporum | | | |
| | Standard A | 100 | II |
| | Verbindungen 1, 14 | 50 | |
| 8) Gibberella avenacea | | | |
| | Standard A | 100 | II |
| | Verbindung 1 | 10 | |
| 9) Gibberella pulicaris | | | |
| | Standard A | 100 | II |
| | Verbindung 1 | 50 | |
| 10) Monographella nivalis | | | |
| | Standard A | 80 | II |
| | Verbindungen 1, 6, 9 | 25 | |
| 11) Phaeosphaeria nodorum | | | |
| | Standard B | 50 | V |
| | Verbindungen 1, 4, 5, 16, 19, 20 | 2, 5 | |
| | Verbindungen 2, 3, 7, 14, 16 | 5 | |
| | Verbindungen 6, 13, 15 | 10 | |
| 12) Pseudocercosporella herpotrichoides | | | |
| | Standard A | 10 | II |
| | Verbindung 13 | 10 | |
| 13) Pyrenophora teres | | | |
| | Standard A | 10 | V |
| | Verbindungen 3, 4, 5, 6, 7 | 1 | |
| | Verbindungen 1, 27 | 10 | |
| 14) Venturia inaequalis | | | |
| | Standard B | 10 | VI |
| | Verbindungen 1, 15, 17, 24 | 2, 5 | |

Beispiel B

Protektive Wirkung der Verbindung gegen Echten Mehltau (Erysiphe cichoracearum) auf Gurken

10 Tage alte Gurkenpflanzen wurden mit einer wäßrigen Verdünnung des formulierten Wirkstoffes besprüht.
Nach dem Antrocknen des Spritzbelages wurden die Pflanzen mit Konidiosporen von befallenen Gurkenpflanzen inokuliert. Die behandelten Pflanzen blieben anschließend im Gewächshaus unter gegebenen Bedingungen stehen.
14 Tage nach der künstlichen Infektion wurde der Befall mit Erysiphe cichoracearum ausgewertet.
Dabei konnten beispielsweise die Verbindungen 1 und 13 in einer Wirkstoffkonzentration von 50 bis 100 ppm den Ausbruch der Krankheit vollständig verhindern.

Beispiel C

Protektive Wirkung der Verbindungen gegen Getreidemehltau (Erysiphe graminis) auf Weizen und Gerste

Gersten- und Weizenpflanzen im frühen 2-Blattstadium wurden mit einer wäßrigen Verdünnung des formulierten Wirkstoffes bis zur Tropfnässe besprüht. Nach dem Antrockenen der Spritzflüssigkeit wurden die Pflanzen mit Konidiosporen von befallenen Pflanzen inokuliert. Die behandelten Pflanzen blieben anschließend im Gewächshaus unter gegebenen Bedingungen stehen.
8 bis 10 Tage nach der künstlichen Infektion wurde der Befall mit Erysiphe graminis ausgewertet.
Dabei konnten beispielweise die Verbindungen 1, 13, 24 und 25 in einer Wirkstoffkonzentration von 100 ppm den Ausbruch der Krankheit vollständig verhindern.

Beispiel D

Protektive Wirkung der Verbindungen gegen Weizenbraunrost (Puccinia recondita) an Weizen

Weizenpflanzen im frühen 2-Blattstadium wurden mit einer wäßrigen Verdünnung des formulierten Wirkstoffes bis zur Tropfnässe besprüht. Nach dem Antrockenen der Spritzflüssigkeit erfolgte die Inokulation mit von infizierten Pflanzen gewonnenen Uredosporen. Anschließend wurden die Testpflanzen für 24 Stunden bei 20 °C und ca. 95 % Luftfeuchtigkeit in einem Klimaraum inkubiert.
Bis zum vollen Krankheitsausbruch auf den Kontrollpflanzen standen die Testpflanzen unter gegebenen Bedingungen im Gewächshaus.
In dieser Prüfung verhinderten beispielsweise die Verbindungen 1, 2, 7, 10 und 13 in einer Konzentration von 50 bis 200 ppm vollständig den Befall der Testpflanzen.

Beispiel E

Protektive Wirkung der Verbindungen gegen Haferkronenrost (Puccinia coronata) an Hafer

Haferpflanzen im frühen 2-Blattstadium wurden mit einer wäßrigen Verdünnung des formulierten Wirkstoffes bis zur Tropfnässe besprüht. Nach dem Antrocknen der Spritzflüssigkeit erfolgte die Inokulation mit von infizierten Pflanzen gewonnenen Uredosporen. Anschließend wurden die Testpflanzen für 24 Stunden bei 20 °C und ca. 95 % Luftfeuchtigkeit in einem Klimaraum inkubiert.
Bis zum vollen Krankheitsausbruch auf den Kontrollpflanzen standen die Testpflanzen unter gegebenen Bedingungen im Gewächshaus.
In dieser Prüfung verhinderten beispielsweise die Verbindungen 1, 10 und 13 in einen Konzentration von 50 bis 200 ppm in ausreichendem Maß den Befall der Pflanzen.

Beispiel F

Protektive Wirkung der Verbindungen gegen Weizenblattdürre (Phaeosphaeria nodorum) an Weizen

Weizenpflanzen im frühen 2-Blattstadium wurden mit einer wäßrigen Verdünnung des formulierten Wirkstoffes bis zur Tropfnässe besprüht. Nach dem Antrocknen der Spritzflüssigkeit erfolgte die Inokulation mit einer wäßrigen Suspension von Pyknidiosporen von Phaeosphaeria nodorum.
Anschließend wurden die Testpflanzen für 36 bis 48 Stunden bei 20 °C und ca. 95 % Luftfeuchtigkeit in einem Klimaraum inkubiert.
Bis zum vollen Krankheitsausbruch auf den Kontrollpflanzen standen die Testpflanzen unter gegebenen. Bedingungen im Gewächshaus.
In dieser Prüfung verhinderten beispielsweise die Vebindungen 1, 6 und 13 in einer Konzentration von 200 ppm a. i. vollständig oder im ausreichenden Maß einen Befall der Pflanzen.

Beispiel G

Protektive Wirkung der Verbindungen gegen Helminthosporiose (Cochliobolus sativus) an Weizen

Weizenpflanzen im frühen 2-Blattstadium wurden mit einer wäßrigen Verdünnung des formulierten Wirkstoffes bis zur Tropfnässe besprüht. Nach dem Antrocknen der Spritzflüssigkeit erfolgte die Inokulation mit einer wäßrigen Suspension von Konidiosporen von Cochliobolus sativus. Anschließend wurden die Testpflanzen 24 Stunden bei 20 °C und ca. 95 % Luftfeuchtigkeit in einem Klimaraum inkubiert.
Bis zum vollen Krankheitsausbruch auf den Kontrollpflanzen standen die Testpflanzen unter gegebenen Bedingungen im Gewächshaus.
In dieser Prüfung verhinderten beispielsweise die Verbindungen 1, 6, und 17 in einer Konzentration von 200 ppm a.i. vollständig oder im ausreichenden Maß einen Befall der Pflanzen.

Beispiel H

Antimykotische Wirkung der Verbindungen gegen Hefen, Schimmelpilze und Dermatophyten

Die Substanzen wurden in DMSO gelöst und mit sterilem Wasser auf verschiedene Konzentrationen (0.19 - 100 µg/ml) verdünnt. Aus diesen Verdünnungen werden jeweils 0.5 ml dem flüssigen Nährmedium zugesetzt.

Die einzelnen Stämme wurden auf Sabouraud-Bierwürze-Schrägagar gewartet und machten vor ihrem Testeinsatz eine Passage auf einem modifizierten Sabouraud-Flüssig-Nährboden durch, wurden anschließend geerntet, gewaschen und zu einer Suspension von McFaerland 3 bei den Hefen und Schimmelpilzen und zu einer Suspension von McFaerland 4 bis 5 bei den Dermatophyten aufbereitet.

Pro Reagenzglas wurden 100 Mikroliter des aufbereiteten Materials inokuliert (Einsaatdichten: Hefen ca. $10^3$/ml, Schimmelpilze und Dermatophyten ca. $10^4$/ml). Der pH-Wert des Flüssignährbodens betrug 6,0. Nach erfolgter Inokulation wurden die Pilze bei 22 °C 14 Tage lang gebrütet.

Zur Bestimmung der antimykotischen Aktivität der Verbindungen wurden der MHK-Wert herangezogen und zwar jene Konzentrationsstufe, bei der makroskopisch kein sichtbares Wachstum mehr festgestellt werden konnte.

Als Vergleichssubstanz diente das 1-(2-(2,4-Dichlorphenyl)-2-((2,4-dichlorphenyl)-methoxy-ethyl)-1H-imidazol-Nitrat) (Verbindung A).

| Ergebnisse | | | | |
|---|---|---|---|---|
| MHK-Werte | (Mikrogramm/Milliliter) | | | |
| Verbindung Nr. | 1 | 7 | 9 | A |
| Trichophyton mentagrophytes | 0,78 | 1,56 | 0,78 | 1,56 |
| Trichophyton rubrum | 0,78 | 0,78 | 0,39 | 6,25 |
| Trichophyton verrucosum | 0,78 | 1,56 | 0,78 | 6,25 |
| Microsporum canis | 0,78 | 6,25 | 3,12 | 6,25 |
| Epidermophyton floccosum | 0,78 | 0,78 | 0,39 | 1,56 |
| Microsporum gypseum | 0,78 | 3,12 | 3,12 | 3,12 |
| Candida albicans | 12,5 | 12,5 | 12,5 | 12,5 |
| Candida tropicalis | 6,25 | 6,25 | 6,25 | 12,5 |
| Aspergillus fumigatus | 0,78 | 12,5 | 25 | 3,12 |
| Mucor mucedo plus | 0,78 | 6,25 | 3,12 | 12,5 |
| Mucor mucedo minus | 0,78 | 6,25 | 3,12 | 12,5 |
| Absidia ramosa | 6,25 | 6,25 | 12,5 | 12,5 |

## Ansprüche

1. Allylaminoethylazole der allgemeinen Formel I

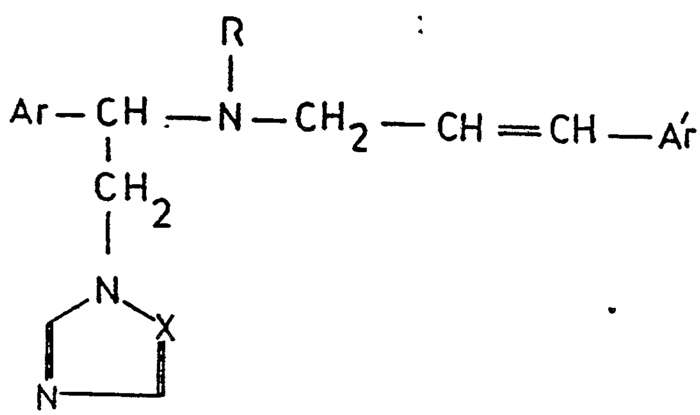

I.

in der
X ein Stickstoffatom oder eine CH-Gruppe
R ein Wasserstoffatom oder eine Alkylgruppe (1 bis 5 C-Atome) und
Ar und Ar′ unabhängig voneinander einen unsubstituierten, ein- oder mehrfach durch Alkyl (1 bis 3 C-Atome), Alkoxy (1 bis 3 C-Atome), Nitrogruppen oder Halogen substituierten Phenylrest, einen unsubstituierten oder durch Halogen substituierten Thienylrest, oder einen Naphthylrest bedeuten, sowie deren pflanzenphysiologisch oder pharmakologisch verträglichen Säureadditionssalze.

2. Allylaminoethylazole der allgemeinen Formel I nach Anspruch 1 in der X eine CH-Gruppe und R ein Wasserstoffatom bedeutet.

3. Allylaminoethylazole der allgemeinen Formel I nach einem der Ansprüche 1 oder 2 in der Ar einen 2,4-Dichlorphenylrest bedeutet.

4. 1-(2-(2,4-Dichlorphenyl)-2-(3-phenylallylamino))ethyl-1H-imidazol

5. Verfahren zur Herstellung von Allylaminoethylazolen der allgemeinen Formel I nach Anspruch 1 in der X, R, Ar und Ar′ die im Anspruch 1 genannte Bedeutung haben, dadurch gekennzeichnet, daß man
a) eine Verbindung der allgemeinen Formel II

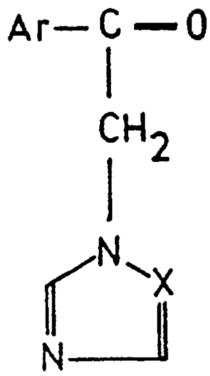

II

in der X und Ar die obgenannte Bedeutung haben, mit einer Verbindung der allgemeinen Formel III
$NH_2$-$CH_2$-CH = CH-A′r III
in der Ar′ die obgenannte Bedeutung hat, gegebenenfalls in einem inerten Verdünnungsmittel zu einer Verbindung der allgemeinen Formel IV

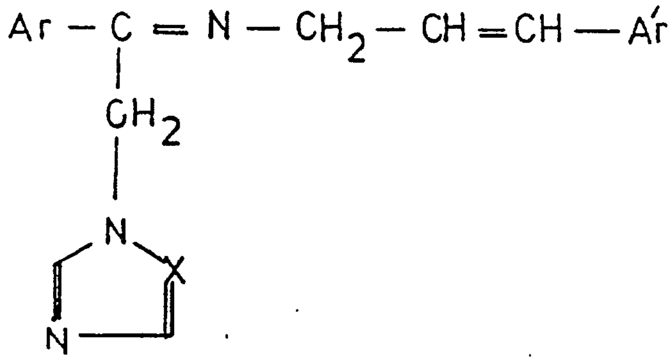

IV

oder
b) eine Verbindung der allgemeinen Formel V

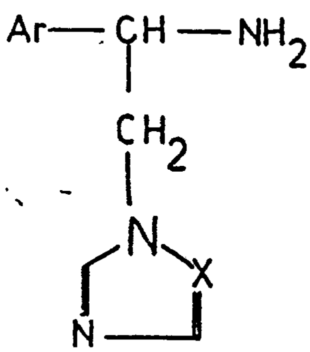

V

in der X und Ar die obgenannte Bedeutung haben mit einer Verbindung der allgemeinen Formel VI

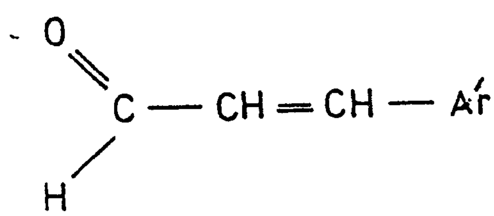

VI

in der Ar' die obgenannte Bedeutung hat gegebenenfalls in einem inerten Verdünnungsmittel zu einer Verbindung der allgemeinen Formel VII

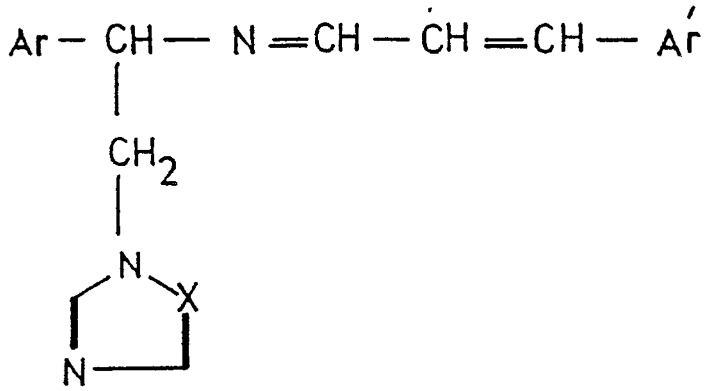

VII

umsetzt
und anschließend die gebildete Verbindung der allgemeinen Formel IV oder die gebildete Verbindung der allgemeinen Formel VII in Gegenwart eines inerten Verdünnungsmittels durch Zugabe eines Reduktionsmittels zu einer Verbindung der allgemeinen Formel I, in der R ein Wasserstoffatom bedeutet, reduziert und gewünschtenfalls zu einer Verbindung der allgemeinen Formel I in der R einen Alkylrest (1 bis 5 C-Atome) bedeutet, alkyliert.

6. Fungizide Mittel gekennzeichnet durch einen Gehalt an mindestens einem Allylaminoethylazol der allgemeinen Formel I nach Anspruch 1.

7. Antimykotisches Mittel, gekennzeichnet durch einen Gehalt an mindestens einem Allylaminoethylazol der allgemeinen Formel I nach Anspruch 1.

8. Verfahren zur Bekämpfung pathogener Pilze, dadurch gekennzeichnet, daß man Allylaminoethylazole der allgemeinen Formel I nach Anspruch 1 auf die Pilze oder deren Lebensraum einwirken läßt.

9. Verwendung von Allylaminoethylazolen der allgemeinen Formel I nach Anspruch 1 zur Bekämpfung pathogener Pilze.

10. Verfahren zur Herstellung fungidizider oder antimykotischer Mittel, dadurch gekennzeichnet, daß man ein Allylaminoethylazol der allgemeinen Formel I nach Anspruch 1 mit Hilfs- und/oder Trägerstoffen vermischt.

Patentansprüche für folgende Vertragsstaaten: GR,ES

1. Verfahren zur Herstellung von Allylaminoethylazolen der allgemeinen Formel I des Formelblattes in der
X ein Stickstoffatom oder eine CH-Gruppe
R ein Wasserstoffatom oder eine Alkylgruppe (1 bis 5 C-Atome) und
Ar und Ar' unabhängig voneinander einen unsubstituierten, ein- oder mehrfach durch Alkyl (1 bis 3 C-Atome), Alkoxy (1 bis 3 C-Atome), Nitrogruppen oder Halogen substituierten Phenylrest, einen unsubstituierten oder durch Halogen substituierten Thienylrest, oder einen Naphthylrest bedeutet, dadurch gekennzeichnet, daß man
a) eine Verbindung der allgemeinen Formel II des Formelblattes in der X und Ar die obgenannte Bedeutung haben, mit einer Verbindung der allgemeinen Formel III des Formelblattes in der Ar' die obgenannte Bedeutung hat, gegebenenfalls in einem inerten Verdünnungsmittel zu einer Verbindung der allgemeinen Formel IV des Formelblattes oder
b) eine Verbindung der allgemeinen Formel V des Formelblattes in der X und Ar die obgenannte Bedeutung haben mit einer Verbindung der allgemeinen Formel VI des Formelblattes in der Ar' die obgenannte Bedeutung hat gegebenenfalls in einem inerten Verdünnungsmittel zu einer Verbindung der allgemeinen Formel VII des Formelblattes umsetzt
und anschließend die gebildete Verbindung der allgemeinen Formel IV des Formelblattes oder die gebildete Verbindung der allgemeinen Formel VII des Formelblattes in Gegenwart eines inerten Verdünnungsmittels durch Zugabe eines Reduktionsmittels zu einer Verbindung der allgemeinen Formel I des Formelblattes, in der R ein Wasserstoffatom bedeutet, reduziert und gewünschtenfalls zu einer Verbindung der allgemeinen Formel I des Formelblattes, in der R einen Alkylrest (1 bis 5 C-Atome) bedeutet, alkyliert.

2. Verfahren nach Anspruch 1, dadurch gekennzeichnet, daß man die Verbindungen der allgemeinen Formel I durch Zusetzen einer Säure gegebenenfalls in einem Lösungsmittel in die pflanzenphysiologisch oder pharmakologisch verträglichen Säureadditionssalze überführt.

3. Verfahren nach Anspruch 1, dadurch gekennzeichnet, daß man eine Verbindung der allgemeinen Formel II oder eine Verbindung der allgemeinen Formel V des Formelblattes, in der X eine CH-Gruppe bedeutet, einsetzt.

4. Verfahren nach Anspruch 1, dadurch gekennzeichnet, daß man eine Verbindung der allgemeinen Formel II oder eine Verbindung der allgemeinen Formel V, in der Ar einen 2,4-Dichlorphenylrest bedeutet, einsetzt.

5. Verfahren nach Anspruch 1, dadurch gekennzeichnet, daß man eine Verbindung der allgemeinen Formel III oder eine Verbindung der allgemeinen Formel VI, in der Ar′ einen 2,4-Dichlorphenylrest bedeutet, einsetzt.

6. Verfahren nach Anspruch 1, dadurch gekennzeichnet, daß man eine Verbindung der allgemeinen Formel III oder eine Verbindung der allgemeinen Formel VI in der Ar′ einen 4-Chlorphenylrest bedeutet, einsetzt.

7. Verfahren nach Anspruch 1, dadurch gekennzeichnet, daß man eine Verbindung der allgemeinen Formel III oder eine Verbindung der allgemeinen Formel VI des Formelblattes, in der Ar′ einen Phenylrest bedeutet, einsetzt.

8. Verfahren zur Herstellung eines Mittels zur Bekämpfung pathogener Pilze, dadurch gekennzeichnet, daß man mindestens ein Allylaminoethylazol der allgemeinen Formel I nach Anspruch 1 oder ein pflanzenphysiologisch oder pharmakologisch verträgliches Säureadditionssalz davon mit üblichen Träger-und Hilfsstoffen vermischt.

9. Verwendung eines Mittels, das mindestens ein Allylaminoethylazol der allgemeinen Formel I nach Anspruch 1 oder ein pflanzenphysiologisch oder pharmakologisch verträgliches Säureadditionssalz davon neben üblichen Träger- und Hilfsstoffen enthält, zur Bekämpfung pathogener Pilze.

10. Verfahren zur Bekämpfung pathogener Pilze, dadurch gekennzeichnet, daß man ein Mittel, das mindestens ein Allylaminoethylalzol der allgemeinen Formel I nach Anspruch 1 oder ein pflanzenphysiologisch oder pharmakologisch verträgliches Säureadditionssalz davon neben üblichen Träger-und Hilfsstoffen enthält, auf die Pilze oder deren Lebensraum einwirken läßt.

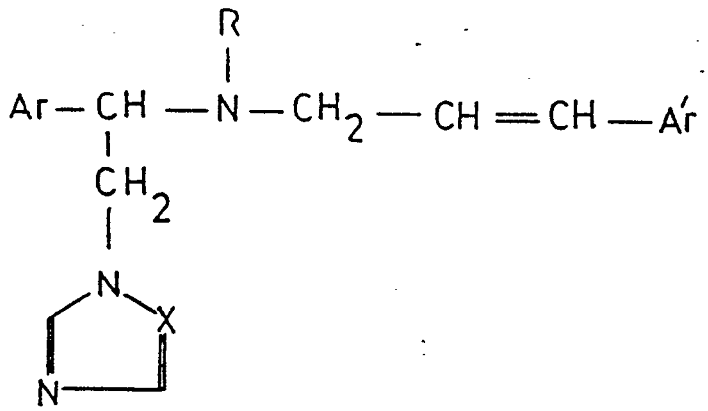

I

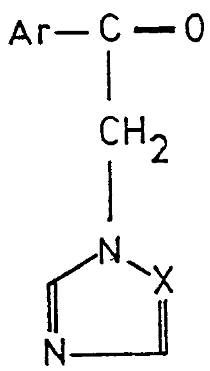

II

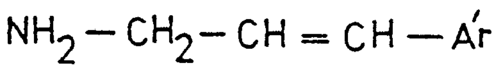

III

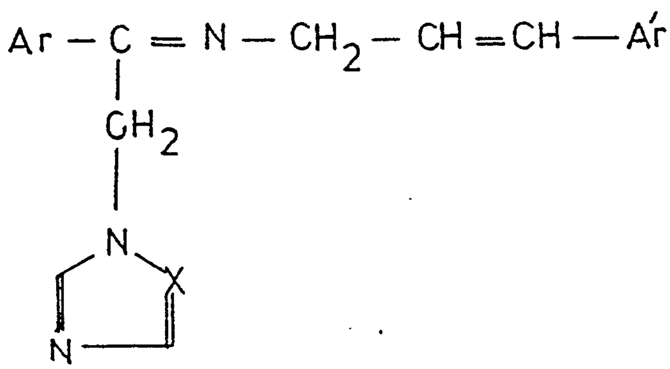

IV

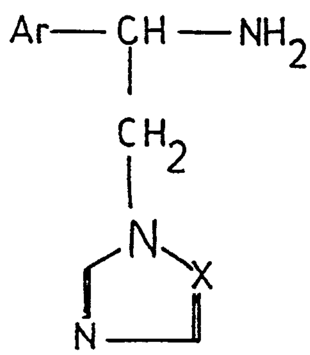

V

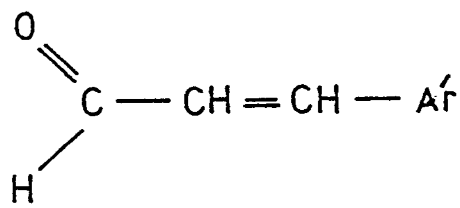

VI

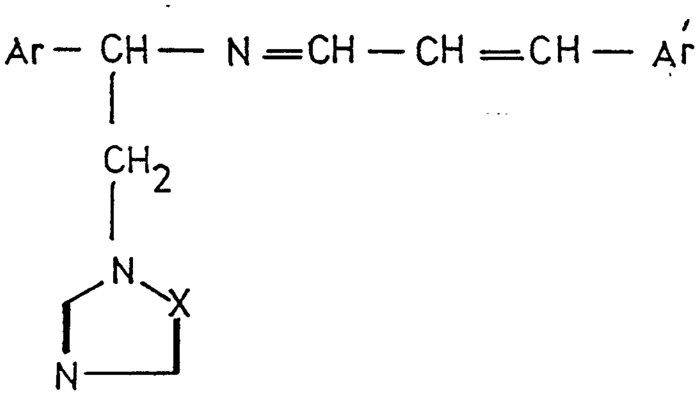

VII